Europäisches Patentamt

European Patent Office

Office européen des brevets

(11) Publication number: **0 300 102 B1**

# EUROPEAN PATENT SPECIFICATION

(45) Date of publication of patent specification: **24.03.93**

(51) Int. Cl.⁵: **A61K 39/00**, //A61K9/52, A61K39/40,A61K37/02, A61K35/20

(21) Application number: **87306455.4**

(22) Date of filing: **21.07.87**

(54) **Improved method of obtaining immune regulatory factors by mammal immunization.**

(43) Date of publication of application:
**25.01.89 Bulletin 89/04**

(45) Publication of the grant of the patent:
**24.03.93 Bulletin 93/12**

(84) Designated Contracting States:
**AT BE CH DE FR GB IT LI LU NL SE**

(56) References cited:
**EP-A- 0 064 103**
**WO-A-85/03635**
**FR-A- 2 439 014**
**GB-A- 2 013 691**

**CHEMICAL ABSTRACTS, vol. 103, no. 18, 14 November 1985, Columbus, OH (US); P.ARTURSSON et al., p. 331, no. 147049b**

(73) Proprietor: **THE STOLLE RESEARCH AND DE-VELOPMENT CORPORATION**
**6990 Cornell Road**
**Cincinnati Ohio 45242(US)**

(72) Inventor: **Beck, Lee R.**
**2550 Dunmore Place**
**Birmingham, AL 35226(US)**

(74) Representative: **Burford, Anthony Frederick**
**W.H. Beck, Greener & Co. 7 Stone Buildings**
**Lincoln's Inn**
**London WC2A 3SZ (GB)**

Rank Xerox (UK) Business Services
(3.10/3.5x/3.0.1)

**EP 0 300 102 B1**

**Description**

The present invention relates to a method of immunizing mammals by inoculation with antigenic substances. More particularly, the invention relates to an improved method of inoculating bovid animals in such a way as to achieve higher antibody titer and lymphokinen levels than can be achieved by conventional inoculation procedures.

Through recent studies, it has been found that the milk taken from a bovid animal such as a cow which has been treated with a spectrum of bacterial antigens is useful in alleviating or eliminating some pathogenic conditions when ingested by a host subject. By virtue of the high antibody titer or the lymphokinen levels of the milk, a subject who ingests the milk or product derived from the milk increases the antibody or lymphokinen count of his own system thereby being able to more effectively resist and defeat a given pathological condition(s) against which the given immune regulatory factors are effective.

For example, US-A-4,284,623 and EP-A-0064103 disclose a method of treating inflammation in a host subject by way of the host subject, exhibiting an inflammatory condition, ingesting milk or a product derived therefrom which has been obtained from a bovid such as a cow which has been inoculated with a spectrum of antigens. The antigens can be any combination of bacterial, viral or cellular antigens with the only substantive restrictive factor being that the bovid being inoculated must be able to respond to the antigen challenge by exhibiting a state of immune sensitivity. This state of immune sensitivity is reflected by a high anti-inflammatory factor or antibody factor in the milk obtained from the animal. The anti-inflammatory factor or immune regulatory factor is a product of the sensitized lymphocyte, more generally referred to as a lymphokinen. The milk obtained in this fashion is capable of alleviating inflammation in a subject who ingests the milk or product derived from said milk.

US-A-4,324,782 describes another example of the therapeutic effectiveness of an antibody containing bovid milk. Cows, immunized with one or more strains of Streptococcus mutans antigen, produce an antibody containing milk, which, when ingested by a host subject, provides the host subject with antibodies effective against the growth of S. mutans bacteria in the oral cavity thereby aiding in the prevention of tooth decay.

Other prior art procedures are known for producing milk which have a variety of therapeutic effects. Heinbach, US-A-3,128,230 has described milk containing globulins of $\alpha$, $\beta$, $\gamma$ components, by inoculating a cow with antigenic mixtures. Petersen (US-A-3,376,198 and CA-A-587,849), Holm, U.S. application (published) Serial No. 628,987 and Tunnak et al, GB-A-1,211,876 have also described antibody-containing milks.

WO-A-8503635 discloses the immunization of a mammal by implantation of an antigen-carrying substrate, preferably in the peritoneal cavity. The antigen-carrying substrate is prepared by dissolving an antigen in a solvent therefor and impregnating a porous biologically-compatible substrate, especially nitrocellulose, with the resulting solution.

All of the above known procedures for obtaining antibody-containing milk are disclosed as being obtained by standard immunization procedures well-known to skilled veterinarians. Usually, a bovid animal is immunized by an initial injection of antigen-containing fluid with the initial injection being followed at specific time intervals by booster injections in order to raise the antibody titer of the bovid to an immune state. If, after a first series of booster injections, the animal is not in a sufficiently immune state, the animal must be inoculated with a second series of injections in order to reach the desired level of immunity.

In view of the problems associated with inoculating a subject bovid animal to obtain an antibody titer level in the animal which is sufficient for the purposes desired with respect to frequency of inoculation and the titer levels which can be achieved, a need continues to exist for a method by which high antibody and/or lymphokinen levels in a bovid animal can be achieved with minimal injections of antigenic material to reach the desired titer levels.

Accordingly, one object of the present invention is to provide a method of achieving an immune state in a mammal, particularly a bovid animal, with fewer inoculations of a given antigenic substance(s) than required by conventional immunization procedures.

Another object of the present invention is to provide a method of producing antibodies and lymphokinen factors in milk.

Briefly, this object and other objects of the present invention as thereinafter will become more readily apparent can be attained in a method of placing a mammal in an immunized state by administering an amount of an antigenic substance (hereinafter "antigen") sufficient to elicit an immunization response, the antigenic substance being administered in the form of microparticles of a biocompatible matrix material. In an alternative embodiment of the method the immunized state is attained more rapidly by simultaneously administering said microparticles to the mammal and inoculating the mammal with liquid antigen.

2

EP 0 300 102 B1

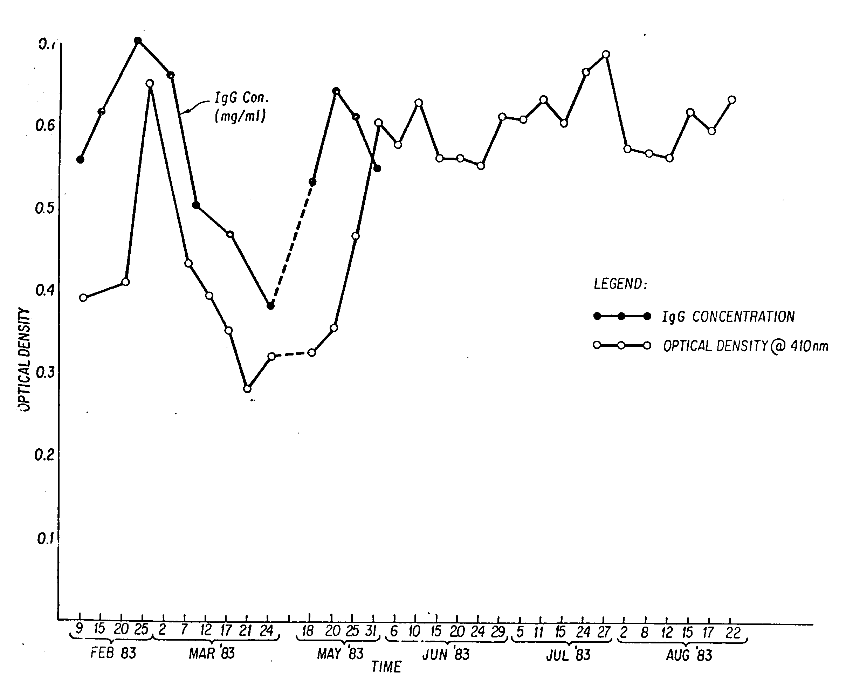

A more complete appreciation of the invention and many of the attendant advantages thereof will be readily obtained as the same becomes better understood by reference to the following detailed description when considered in connection with the accompanying drawing, wherein:

the FIGURE is a graph in which optical density and IgG titer level are plotted as a function of time for cattle which have been immunized against the S-100 spectrum of bacterial antigens.

The heart of the present invention is the discovery that, when a mammal is implanted with microparticles containing at least one antigen, an immunization response can be attained with as few as only one treatment of microparticles. An additional and unexpected aspect of the discovery is that by the use of the microparticles, antibody titer and lymphokinen levels greater than normally reached by conventional methods of inoculation or injection can be achieved.

Any mammal can be treated with an antigen by the technique of the present invention. A preferred group of mammals are the bovids which include any milk-producing member of the genus Bos, preferably cows, sheep and goats, most preferably cows.

The microparticles used in the present invention can be generally formed from any polymeric material which is biocompatible preferably and biodegradable or bioerodible. The term "biocompatible" means that the matrix material must be compatible with the tissues of the mammal and more particularly the muscle tissues of the mammal in that it does not result in inflammation of the tissues and must not itself cause an immune reaction. The term "biodegradable" means that the matrix material of the microparticles must be easily broken down by body processes into smaller molecular units which are readily disposed of or are easily oxidized by the body. The molecular units themselves, just like the macromolecular matrix material, must also be biocompatible. If the matrix material is not biodegradable, it must be at least bioerodible, or otherwise permeable porous to allow release of the encapsulated antigen into the body.

Because the delivery system of the present invention is a solid, shaped mass of matrix material and not a fluid medium, once the microparticles are administered to a mammal, the encapsulated antigen is not immediately released to the body, as is the case for antigens in a liquid vehicle, but rather the antigen is slowly released in a controlled fashion over extended periods of time. The rate at which antigens are released to the system is dependent upon the matrix material employed, its porosity, the rate at which the matrix material degrades or erodes among other factors. Of course, each individual matrix material exhibits its own characteristic rate of release. The rate of antigen release must be fast enough for the body to exhibit an immune response, and not too fast such that most or all of the encapsulated antigen is released within a very short period. The release of antigen from the microparticles may occur by diffusion of the agent from the matrix material or by erosion of the matrix material or by a combination of both factors.

Suitable polymeric matrix materials from which the microparticles can be formed include aliphatic polyesters, polyorthoesters, polyanhydrides, polyamides, polycyclic thioethers, copolyoxalates, polyvinyl alcohol, polypeptides and polydioxanones. Preferred polymeric matrix materials include polylactic acid, polyglycolic acid, poly(lactic acid-co-glycolic acid), polycaprolactone, copolyoxalates, and ethylene vinyl/acetate copolymers. Other suitable polymeric materials include proteins such as collagen, fatty acid esters such as the glycerol stearates and cellulose esters such as cellulose acetate butyrate.

In preparing the microparticles, compositions of different rates of controlled release can be prepared. One suitable method of formulation is to incorporate a single antigen or a mixture of at least two antigens into a single matrix. This process is repeated one or more times with a different matrix material having a different rate of release characteristic from that of the previously employed matrix material. Finally, the microparticles are blended together to prepare an antigen releasing formulation having a range of release rates. Alternatively, microparticles can be prepared by incorporating a single antigen or mixture of antigens in a blend of two or more different matrix materials to prepare a product in which each microparticle is made up of a blend of the matrix materials used in the preparation.

Preferably the microparticles contain the antigen incorporated throughout the matrix material of the microparticles. The microparticles can be of any shape since shape is not a critical factor associated with release of the entrapped antigen. Generally, however, the microparticles will be spherical in shape. In a preferred method of production spherically shaped microparticles are prepared and the microspheres are between 1 to 500, preferably 10 to 200, micrometers in diameter. The rate of release of antigen from the microparticles is related to size in some formulations.

In order to place the microparticles in administratable form, the microparticles are placed in an appropriate liquid vehicle for injection into the body such as a saline solution containing a small amount of surfactants.

The molecular weight of the particular matrix material chosen is not a critical factor in the manufacture and use of the microparticles. While an increase in the molecular weight of the polymer may gradually retard the rate of release of antigen from the matrix material, these factors may easily be compensated for

3

by determining the rate of release of antigen from the matrix material by in vivo or in vitro measurements and then adjusting the amount of microparticles administered and the other design parameters such as microparticle size and percent by weight of the antigen in view of the results obtained from the release measurements.

In the manufacture of the microparticles, any conventional method of forming the microparticles can be used. The selection of a particular method chiefly depends upon the technical requirements of the matrix material and the particular manner in which the microparticles are intended to be used.

Generally, microencapsulation processes can be classified according to the three principal types of: (1) phase-separation methods including aqueous and organic phase separation processes, melt dispersion and spray drying; (2) interfacial reactions including interfacial polymerization, in situ polymerization and chemical vapor deposition: and (3) Physical methods, including fluidized-bed spray coating, multi- and single-orifice centrifugal coating, electrostatic coating and physical vapor deposition.

Phase separation methods, as the term implies, rely on differential solubility characteristics that cause a wall- or shell-forming matrix material to separate from solution or suspension and deposit around particles or droplets of the antigen to be encapsulated. The separation, itself, may be brought about physically, as by the addition of a non-solvent or by a change in temperature, or chemically, as by a change in pH.

An organic phase-separation process usually employs a dispersion or an emulsion of the antigen in a solution or a high-molecular-weight polymer in an organic solvent. To this mixture is added a non-solvent or liquid polymer that causes the high-molecular-weight polymer to separate from solution and collect as a shell around the suspended antigen. The shell, still swollen with solvent, is then hardened by a further addition of non-solvent or by some other process that strengthens the shell and improves the barrier properties.

Typically, an aqueous solution or suspension of a lipophobic antigen is added to a non-aqueous solution of a suitable matrix polymer, and the mixture is agitated to cause the formation of a water-in-oil emulsion. Depending upon its solubility in water, the agent may be present at a concentration of 5 to 50% in the aqueous phase, which may be 5 to 20% by weight of the total mixture. The external organic phase may contain 5 to 10% of the matrix polymer. Usually, however, the ratio of agent in the internal phase (aqueous solution or suspension) to polymer is 2:1 to 1:4. The polymer must be a good film-former: that is, it must possess adequate strength and toughness.

An aqueous phase separation process employs a dispersion or an emulsion of a water-insoluble antigen in an aqueous solution or dispersion of a polymer. The polymer is caused to separate as gel particles; these collect around the antigen to form a shell; the shell is hardened; and the microparticles are isolated. In the coacervation process, which is the most common of the aqueous phase-separation processes the water-insoluble antigen, which may be in the form of particles or droplets, is usually dispersed in an aqueous sol of a hydrophilic colloid which becomes ionized in water; a second sol of opposite charge is added; and the mixture is caused to gel by a dilution with water, an addition of salt, an adjustment of pH, or a change in temperature, or any combination of these procedures. Appropriate conditions of coacervation are usually determined experimentally, because the various polymers, possible for use, differ significantly in physical and chemical properties according to source and method of isolation or preparation. A region of coacervation is determined by combining solutions or sols of two polymers at various concentrations, temperatures and levels of pH, and observing the conditions required for gelation. From these determinations can be drawn a ternary phase diagram, showing the area of compatibility and the region of coacervation, at a given temperature and pH. The changes in concentration, temperature or pH to effect gelation will then become apparent.

Each preparation of microparticles requires careful control of conditions, and somewhat different conditions are required for various materials being encapsulated. The degree of agitation, for example, affects the size of emulsion droplets, and the surface properties of the droplets may require alterations in the procedures to insure deposition of matrix material about the droplets and to minimize formation of particles not participating in microencapsulation. The volume of water added in the dilution step is not critical, but generally larger volumes are required to maintain a stable emulsion when larger droplets are encapsulated.

The above phase separation can be adapted to an alternate technique in which the first step of forming a stable emulsion or suspension of a substance is accomplished by dispersing the antigen in a solution of the matrix material. Thereafter, the emulsion is added dropwise to a non-solvent with stirring to precipitate the polymer coating material to form microparticles.

Another type of phase separation technique is the melt-dispersion microencapsulation technique. This method can be used with a wide variety of substances to be encapsulated. Usually a heat-liquefiable, waxy coating material, preferably of a low-melting wax such as glycerol distearate is suspended in an inert liquid such as a silicone oil or a fluorocarbon in which neither the wax nor the antigen to be encapsulated is appreciably soluble. The mixture is heated and stirred vigorously to melt and emulsify the wax. The antigen, which has been powdered and screened to the desired size range, and the waxy coating material are dispersed with high shear agitation, and the liquefied wax coats the antigen to form the waxy liquid-coated microparticles. Thereafter, the formed microparticles are solidified by continued agitation which cools the particles. The microparticles are then isolated by filtration and dried as described earlier.

The second major method of forming the microparticles is by interfacial microencapsulation which involves bringing two reactants together at a reaction interface where polycondensation of the reactants, usually monomers, occurs to form a thin, insoluble polymeric film. One technique of establishing the interface for the encapsulation process is the dispersion or emulsification of the antigen with one of the reactants which form the condensation polymer in a continuous phase containing the second reactants.

The third major category of encapsulation techniques which is especially applicable to a variety of antigens and coating materials is physical microencapsulation. The physical microencapsulation techniques are characterized by the continuous envelopment of particles or droplets of antigen in a fluid film, as a melt or solution of the coating material, in an apparatus containing coaxially or sequentially-spaced orifices. Thereafter, the fluid coating is hardened by a standard cooling technique or by solvent evaporation.

Among the physical methods for microencapsulation are those that involve the passage of liquid or solid antigen core material through a liquid matrix material. The stream is disrupted by some means to cause the formation of liquid-coated droplets or particles, and the resulting particles are cooled or otherwise treated to solidify the shell material. For example, an aqueous solution of antigen to be encapsulated is aspirated into rapidly flowing stream of molten glycerol distearate, and the mixture is ejected through a fine nozzle. On emergence from the nozzle, the liquid stream disintegrates into droplets, each consisting of an aqueous core surrounded by liquid wax. As these fall through air, the shells cool and solidify, and microparticles result. In another version of this process, the impelling force is supplied by a rotating member, which ejects the core material centrifugally through the shell-forming liquid.

The variations of these and other processes of microencapsulation are many. As is readily apparent to those skilled in the art, no one process nor any single set of conditions is applicable to all substances, but instead a useful process is chosen and the conditions optimized to achieve the desired results with a specific antigen.

In a preferred method of preparing microparticles containing an antigen, a phase separation technique is employed whereby a solution of the polymeric matrix material in a suitable organic solvent is prepared. To this solution is added the antigen suspended or dissolved in water or as fine particles alone. A non-solvent for the polymeric matrix material is slowly added to the stirred dispersion causing the polymeric material to slowly precipitate around the antigen forming microparticles. The microparticles are further hardened by the addition of a second non-solvent for the polymeric matrix material. The microparticles are then isolated by filtration and dried.

The most expedient mode of administration of the microparticles is by intra-muscular injection, although microparticles can be subcutaneously implanted.

The desired objective of the present invention is to bring a mammal into a hyperimmune state, as well as to produce antibodies and lymphokinen factors in milk. By conventional administration procedures this state is usually achieved by an initial inoculation of a mammal with an antigen or mixture of antigens in a liquid vehicle, followed by several booster injections of sufficiently high doses of antigen or mixtures of antigens which are necessary to reach a hyperimmune state. Normally, booster injections are used at two-week intervals to reach and maintain high antibody titer levels in the body fluids of a mammal. It is only when the mammal is in a hyperimmune state that the body fluids of the mammal will contain a sufficiently high antibody titer. In the case of a bovid such as a cow, the milk of a hyperimmunized cow will have a high level of immune regulatory factor which makes the milk useful for a variety of different purposes.

The advantage of the present invention is that by a single implantation of an antigen containing microparticle, not only are booster injections of the antigen not needed to reach a hyperimmune state, but quite unexpectedly and even more importantly, higher levels of immune regulatory factors including antibodies and lymphokinens within the mammal and in the milk of cows can be obtained in comparison to conventional inoculation methods. Because in the present technique the antigen is dispersed throughout the matrix material, it is gradually released from the matrix material thereby providing a sustained, continual release of antigen to the treated mammal. This mode of administration of antigen is quite different from inoculations of antigen where a given quantity of antigen is administered instantaneously followed by depletion of the level of antigen in the body of the mammal until the next inoculation with antigen. For this reason it is very difficult to state just how much antigen is administered to a mammal being immunized by the method of the present invention. Perhaps a given mammal may be administered antigen at a rate of 0.01 to 1.0 mg per day. It may be that the unexpectedly high immune regulatory factor levels achieved by the method of the present invention are attributable to the fact that antigen levels are maintained at relatively constant levels for extended periods of time within the body tissues of the mammal being treated. It is apparent that whatever specific mode the shaped, antigen containing matrix material of the present invention is administered, the delivery system must contain enough antigen to maintain a continued level of antigen for a time sufficient to reach a hyperimmune state. For example, approximately $10^9$ killed bacterial cells can be used in a normal dose per injection, but this number certainly can vary with the type of antigen used.

In another embodiment of the present invention, even higher antibody titers can be obtained by a modification of the present implantation method wherein simultaneously with the implantation of a microparticle, the subject mammal is administered unencapsulated antigen. In other words, the subject mammal at the time of implantation can be inoculated in some manner with unencapsulated antigen, there placing the subject mammal in the state of an immediate high antigen level. This procedure results in the desirable effect of providing higher antibody titers in the subject animal more rapidly than the rate at which high titer levels can be reached only by the basic implantation technique. In the embodiment of the invention where microparticles are being subcutaneously or intramuscularly injected, antigenic substance in a liquid vehicle can be inoculated right along with the microparticles or at another site on the subject mammal. When the microparticles are implanted in a subject animal, unencapsulated antigen may be implanted with the microparticles or it can be inoculated into the subject animal at a different site.

It is necessary to determine whether or not the inoculated mammal has become sensitive to the antigen. There are a number of methods known to those skilled in the art of immunology to test for sensitivity, (Methods in Immunology and ImmunoChemistry, William, C. A., Chase, W. M., Academic Press, N.Y., London, Vol. 1-5, 1977). Examples of these tests include skin sensitivity tests, serum tests for the presence of antibodies to the stimulating antigens and tests designed to evaluate the ability of immune cells from the host to respond to the antigen. The type of test employed will depend to a large extent on the nature of the antigen used. The preferred method is to use a polyvalent vaccine consisting of multiple bacterial species as the antigen and to test for the presence of agglutinating antibodies in the serum of the mammal before and after challenge with the vaccine. In the case of a bovid, the appearance of milk antibodies after immunization with the vaccine is indicative of sensitivity, and at this point the antibody-containing milk produced by the bovid can be utilized for the purpose desired.

Any specific antigen or combination of antigens may be employed in the microparticles. The antigens can be bacterial, viral, cellular or any other substance to which the immune system of the mammal will respond. Preferably, the antigen or mixtures of antigens are bacterial or viral with polyvalent antigens also being preferred. Suitable bacterial antigens include Neisseria qonorrhea, Mycobacterium tuberculosis, Haemophilus vaginalis, Group b Streptococcus ecoli, Microplasma hominis, Staphylococcus aureus, Staphylococcus epidermidis, Streptococcus pyrogenes, Streptococcus mutans, Aerobacter aerogenes, Escherichia coli, Salmonella enteritidis, Pseudomonas aeruginosa, Klebsiella pneumoniae, Salmonella typhimurium, Haemophilus influenzae, Streptococcus viridans, Proteus vulgaris, Shigella dysenteriae, Streptococcus Group B, Diplococcus pneumoniae, Corynebacterium Acne Types 1 and 2, Hemophilus ducreyi, Granuloma inguinale, Lymphopathia venereum, Treponema pallidum, Brucella abortus, Brucella melitensis, Brucella suis, Brucella canis, Campylobacter fetus, Campylobacter fetus intestinalis, Leptospira pomona, Listeria monocytogenes, Brucella ovis, Chlamydia psittaci, Escherichia coli, Actinobacillus equuli, Salmonella abortus ovis, Salmonella abortus equi, Corynebacterium equi, Corynebacterium pyogenes, Actinobaccilus seminis, Mycoplasma Bovigenitalium, Clostridium tetani, Pseudomonas maltophiia, Streptococcus equisimili, Streptococcus dysgalactiae, Streptococcus uberis, Streptococcus bovis, Pasteurella multocida, Pasteurella hamemolytica, Moraxella bovis, Actinobacillus lignieresi, Corynebacterium renale, Fusobacterium necrophorum, Bacillus cereus, Salmonella dublin, Salmonella heidleberg, Salmonella paratyphi, and Yersinia enterocolitica. Suitable viral antigens include Equine herpes virus, Equine arteritis virus, IBR - IBP virus, BVD -MD virus and Herpes virus (humonis types 1 and 2).

The method of the present invention can be used to produce a high level of immunity in a mammal. Accordingly, milk-producing bovid animals can be hyperimmunized by the present technique to produce the antibody and lymphokinen containing milk. The lymphokinen factors are effective against inflammations, as described in US-A-4,284,623, and other conditions which are debilitating cardiovascular and pulmonary conditions as described in US-A-4,636,384. The method of the present invention can also be used to produce the specific antibody-containing milk which is effective against the bacteria which promote dental caries formation, plaque formation and gingivitis as described in US-A-4,324,782 or to produce the antibody-containing milk which is effective against odor and disease causing skin bacteria.

Having generally described this invention, a further understanding can be obtained by reference to certain specific examples which are provided herein for purposes of illustration only and are not intended to be limiting unless otherwise specified.

Preparation of S-100 Vaccine

A bacterial culture containing the spectrum of bacteria shown in Table 1 below as obtained from the American Type Culture Collection was reconstituted with 15 ml of media and incubated overnight at 37°C. Once good growth was obtained, approximately one-half of the bacterial suspension was employed in inoculate one liter of broth with the inoculate being incubated at 37°C. The remaining suspension was transferred to sterile glycol tubes and stored at -20°C for up to six months.

After good growth was visible in the liter culture, the bacterial cells were harvested by centrifugation of the suspension for twenty minutes to remove the media. The bacterial pellet obtained was resuspended in sterile saline solution and the bacterial sample was centrifuged three times to wash the media from the cells. After third sterile saline wash, bacterial pellet obtained upon centrifugation was resuspended in a small amount of double distilled water.

The media-free bacterial suspension was heat-killed by placing the suspension in a glass flask in an 80°C water bath overnight. The viability of the bacteria in the sample was determined by inoculation of the broth culture with a small amount of the heat-killed bacteria. The broth was incubated at 37°C for five days and checked daily for growth, since the bacteria have to be killed for use in the vaccine.

The heat killed bacteria were lyophilized until dry. The dry bacteria were then mixed with sterile saline solution to a concentration of $2.2 \times 10^8$ bacterial cells/ml saline (1.0 optical density reading at 660 nm).

## Table 1

## S-100 Bacteria List

| | Name | Media | Gram + or - | ATCC # |
|---|---|---|---|---|
| 1. | Staph. aureus | BHI | + | 11631 |
| 2. | Staph. epidermidis | BHI | + | 155 |
| 3. | Strep. pyogenes, A. Type 1 | APT | + | 8671 |
| 4. | "     "     "     "     3 | APT | + | 10389 |
| 5. | "     "     "     "     5 | APT | + | 12347 |
| 6. | "     "     "     "     8 | APT | + | 12349 |
| 7. | "     "     "     "     12 | APT | + | 11434 |
| 8. | "     "     "     "     14 | APT | + | 12972 |
| 9. | "     "     "     "     18 | APT | + | 12357 |
| 10. | "     "     "     "     22 | APT | + | 10403 |
| 11. | Aerobacter aerogenes | BHI | - | 884 |
| 12. | Escherichia coli | BHI | - | 26 |
| 13. | Salmonella enteritidis | BHI | - | 13076 |

| | | | |
|---|---|---|---|
| 14. Pseudomonas aeruginosa | BHI | – | 7700 |
| 15. Klebsiella pneumoniae | BHI | – | 9590 |
| 16. Salmonella typhimurium | BHI | – | 13311 |
| 17. Haemophilus influenzae | BHI | – | 9333 |
| 18. Strep. mitis | APT | + | 6249 |
| 19. Proteus vulgaris | BHI | – | 13315 |
| 20. Shigella dysenteriae | BHI | – | 11835 |
| 21. Diplococcus pneumoniae | APT | + | 6303 |
| 22. Propionibacter acnes–Actinomyces | + | | 11827 |
| (anaerobe) | Broth | | |
| 23. Strep. sanguis | APT | + | 10556 |
| 24. Strep. salivarius | APT | + | 13419 |
| 25. Strep. mutans | BHI | + | 25175 |
| 26. Strep. agalactiae | APT | + | 13813 |

Example 1

Eight cows identified by number in Table 2 below were given daily injections of 5 ml samples of the polyvalent liquid vaccine. Antibody (IgG) titer levels for the injected cattle were determined periodically. The antibody (IgG) titer levels were determined by using an enzyme linked immuno-assay (ELISA) system, the end point being an optical density reading at 410 nm of antibody containing fluid samples obtained from cow's milk, the individual reading obtained from each cow being shown in Table below. The higher the optical density reading, the higher the titer. The table also shows the average optical density reading determined from the individual optical density values obtained. The average IgG antibody titer level as determined from the average optical density value for each measurement period is also shown in Table 2. The FIGURE is a graph of the results shown in Table 2.

8

Cow No.                 Sample Date

| | 2/9/83 | 2/15/83 | 2/20/83 | 2/25/83 | 3/2/83 | 3/7/83 | 3/12/83 | 3/17/83 | 3/21/83 | 3/24/83 | 5/18/83 |
|---|---|---|---|---|---|---|---|---|---|---|---|
| 2245 | 0.352 | 0.329 | 0.391 | 0.544 | 0.646 | 0.512 | 0.421 | 0.383 | 0.333 | 0.328 | ------ |
| 2033 | 0.410 | 0.304 | 0.343 | 0.614 | 0.522 | 0.399 | 0.360 | 0.409 | 0.307 | 0.306 | 0.332 |
| 1805 | 0.386 | 0.466 | 0.560 | 1.009 | 0.640 | 0.510 | 0.354 | 0.304 | 0.257 | 0.380 | ------ |
| 2195 | 0.365 | 0.279 | 0.405 | 0.504 | 0.493 | 0.433 | 0.348 | 0.265 | 0.227 | 0.232 | 0.295 |
| 1761 | 0.448 | 0.251 | 0.348 | 0.577 | 0.404 | 0.324 | 0.481 | 0.391 | 0.273 | 0.332 | ------ |
| 2345 | ------ | ------ | ------ | ------ | ------ | ------ | ------ | ------ | ------ | ------ | 0.338 |
| 2093 | ------ | ------ | ------ | ------ | ------ | ------ | ------ | ------ | ------ | ------ | 0.429 |
| 2429 | ------ | ------ | ------ | ------ | ------ | ------ | ------ | ------ | ------ | ------ | 0.235 |
| | | | | | | | | | | | |
| | | | | | | | | | | | |
| AVG. | 0.392 | 0.326 | 0.410 | 0.650 | 0.541 | 0.436 | 0.393 | 0.350 | 0.279 | 0.316 | 0.326 |
| | | | | | | | | | | | |
| IgG Conc. (mg/ml) | 0.557 | 0.629 | 0.701 | ------ | 0.657 | 0.501 | ------ | 0.468 | ------ | 0.379 | 0.529 |

Remarks:

Optical density readings at 410 nm.

Injections given daily 2/9/82- 2/22/83 and 5/18/83 to 10/12/83.

EP 0 300 102 B1

9

## TABLE 2

Date:

| Cow No. | Sample Date | | | | | | | | | | |
|---|---|---|---|---|---|---|---|---|---|---|---|
| | 5/20/83 | 5/25/83 | 5/31/83 | 6/6/83 | 6/10/83 | 6/15/83 | 6/20/83 | 6/24/83 | 6/29/83 | 7/5/83 | 7/11/83 |
| 2245 | ---- | ---- | ---- | ---- | ---- | ---- | ---- | ---- | ---- | ---- | ---- |
| 2033 | 0.426 | 0.598 | 0.673 | 0.731 | 0.756 | 0.467 | 0.585 | ---- | ---- | ---- | ---- |
| 1805 | ---- | ---- | ---- | ---- | ---- | ---- | ---- | ---- | ---- | ---- | ---- |
| 2195 | 0.299 | 0.466 | 0.631 | 0.589 | 0.638 | 0.612 | 0.619 | 0.637 | 0.731 | 0.727 | 0.759 |
| 1761 | ---- | ---- | ---- | ---- | ---- | ---- | ---- | ---- | ---- | ---- | ---- |
| 2345 | 0.333 | 0.445 | 0.750 | 0.508 | 0.643 | 0.635 | 0.592 | 0.562 | 0.591 | 0.532 | 0.475 |
| 2093 | 0.441 | 0.531 | 0.618 | ---- | ---- | ---- | ---- | ---- | ---- | ---- | ---- |
| 2429 | 0.276 | 0.286 | 0.356 | 0.466 | 0.478 | 0.539 | 0.449 | 0.464 | 0.503 | 0.534 | 0.562 |
| | | | | | | | | | | | |
| Average | 0.355 | 0.465 | 0.606 | 0.574 | 0.629 | 0.563 | 0.561 | 0.554 | 0.603 | 0.604 | 0.632 |
| IgG Conc. (mg/ml) | 0.640 | 0.612 | 0.543 | ---- | ---- | ---- | ---- | ---- | ---- | ---- | ---- |

Remarks:

TABLE 2

| Cow No. | Sample Date | | | | | | | | |
|---|---|---|---|---|---|---|---|---|---|
| | 7/15/83 | 7/21/83 | 7/27/83 | 8/2/83 | 8/8/83 | 8/12/83 | 8/15/83 | 8/17/83 | 8/22/83 |
| 2245 | ---- | ---- | ---- | ---- | ---- | ---- | ---- | ---- | ---- |
| 2033 | ---- | ---- | ---- | ---- | ---- | ---- | ---- | ---- | ---- |
| 1805 | ---- | ---- | ---- | ---- | ---- | ---- | ---- | ---- | ---- |
| 2195 | 0.581 | 0.654 | 0.550 | 0.636 | 0.613 | 0.663 | 0.740 | 0.671 | 0.712 |
| 1761 | ---- | ---- | ---- | ---- | ---- | ---- | ---- | ---- | ---- |
| 2345 | 0.578 | 0.643 | 0.611 | 0.581 | 0.584 | 0.597 | 0.592 | 0.589 | 0.692 |
| 2093 | ---- | ---- | ---- | ---- | ---- | ---- | ---- | ---- | ---- |
| 2429 | 0.637 | 0.688 | 0.877 | 0.489 | 0.498 | 0.425 | 0.518 | 0.504 | 0.474 |
| Average | 0.599 | 0.662 | 0.679 | 0.569 | 0.565 | 0.562 | 0.617 | 0.588 | 0.626 |
| IgG Conc. (mg/ml) | --- | --- | --- | --- | --- | --- | --- | --- | --- |

Date:

Remarks:

## Example 2

Heat-killed bacteria were prepared in the manner described above. The polyvalent antigen sample (S-100) obtained was microencapsulated by a conventional phase separation process to prepare a polyvalent antigen containing microparticle product. The polymeric matrix material employed was biodegradable poly (lactide-coglycolide). The microparticles were less than 250 micrometers in diameter. Approximately 750 mg of microparticles containing 22% (16.5 mg) of polyvalent antigen was then suspended in about 3 cc. of a vehicle (1 wt % Tween 20 and 2 wt % carboxymethyl cellulose in water).

A small group of cattle was selected from a larger herd of cattle. Five of these randomly selected cattle were selected as controls. Four cattle identified as numbers 398, 408, 414 and 400 in Table 3 below were treated by an injection of microparticle containing solution prepared as described above.

The four cows were injected intramuscularly with the polyvalent antigen containing microparticles on April 23, 1982. Microparticle samples B022-41-1 and B022-41-2 were sterilized with 2.0 mRad of gamma radiation, while samples B022-41-3 and B022-41-4 were unsterilized. Antibody (IgG) titer levels were determined periodically from samples of cow's milk obtained from the inoculated cows as well as the control cows, and the data obtained are shown in Table 3.

The results obtained from Examples 1 and 2 show that the technique of the present invention for

## Table 3

### Electroimmunoassay & Microtiter Results of Serum Samples from Microencapsulated vs. Random S-100 Cows. Date 7-13-82

Experimental Design:

| Cow | Antigen | Vehicle |
|---|---|---|
| 408 | B022-41-1 | B022-042-1 |
| 400 | B022-41-2 | B022-42-2 |
| 398 | B022-41-3 | B022-42-3 |
| 414 | B022-41-4 | B022-42-4 |

|  |  | Cows Treated with Microencapsulated Antigen 2 |  |  |  |  | Control Cows 1 |
|---|---|---|---|---|---|---|---|
| Cow No. Date of Sample |  | 398 | 408 | 414 | 400 | Mean | Mean |
| I_qG Level (mg/ml) | 4-29-82 | 18.9 | 24.2 | 29.1 | 28.2 | 25.1 | 26.6 |
|  | 5-13-82 | 19.2 | 22.0 | 46.5 | 36.6 | 31.1 | 28.7 |
|  | 6-2-82 | 26.7 | 25.7 | 41.2 | 39.4 | 33.3 | 32.8 |
|  | 6-16-82 | 31.0 | 27.0 | 39.7 | 39.1 | 34.2 | 33.5 |
|  | 6-30-82 | 27.6 | 32.9 | 39.1 | 31.3 | 32.7 | 31.2 |
|  | Mean | 24.7 | 28.0 | 39.1 | 34.9 | 31.3 | 30.6 |
| Micro-liter | 4-28-82 | 376 | 128 | 768 | 512 | 376 | 576 |
|  | 5-13-82 | 608 | 768 | 384 | 768 | 608 | 512 |
|  | 6-2-82 | 608 | 256 | 384 | 768 | 608 | 640 |
|  | 6-16-82 | 1536 | 1536 | 1536 | 1024 | 1536 | 704 |
|  | 6-30-82 | 1472 | 1024 | 2048 | 2048 | 1472 | 536 |
|  | Mean | 890 | 742 | 1024 | 1024 | 920 | 594 |

Note: 1. Five control cows were randomly chosen from the S-100 herd.
2. Microencapsulated cows: 398 calved on 5-28-82
408 calved on 5-27-82
414 calved on 5-15-82
400 calved on 5-20-82

achieving an immune state in an animal, particularly a bovid animal, by fewer inoculations with antigen containing microparticles is superior to conventional methodology of achieving an immune state in an animal by inoculation with conventional antigen containing injectable solutions.

Having now fully described the invention, it will be apparent to one of ordinary skill in the art that many changes and modifications can be made thereto without departing from of the invention.

**Claims**

1. A method of obtaining an immune regulatory factor by immunizing a mammal and collecting or extracting immune regulatory factor-containing substance therefrom, characterised in that the mammal is immunized by implanting within the said mammal an amount of an antigenic substance sufficient to elicit an immunization response, said antigenic substance being incorporated within microparticles of a biocompatible matrix material which causes controlled release of said antigenic substance thereby prolonging antigenic activity within said treated mammal.

2. A method as claimed in Claim 1, wherein said microparticles are implanted in said mammal by intramuscular injection.

3. A method as claimed in Claim 1, wherein said microparticles are implanted in said mammal by subcutaneous injection.

4. A method as claimed in any one of the preceding claims, wherein the matrix material is a biocompatible, biodegradable polymeric matrix material.

5. A method as claimed in Claim 4, wherein said polymeric material is polylactic acid, polyglycolic acid, poly(lactic acid-co-glycolic acid), polycaprolactone, polyoxalic acid, polydioxanone, polyorthoester or the salt form of these polymers.

6. A method as claimed in any one of the preceding claims, wherein the microparticles are formed by incorporating said antigenic substance(s) in a mixture of at least two different biodegradable, biocompatible polymers, each having a different rate of biodegradation, thereby forming individual microparticles composed of a mixture of polymeric matrix materials.

7. A method as claimed in any one of Claims 1 to 5, wherein the microparticles are formed by (a) incorporating said antigenic substance(s) in a single polymeric matrix material, (b) separately repeating the process of step (a) at least once with a different polymeric matrix material having a rate of biodegradation different from the first used matrix material of step (a), and then (c) completing the preparation of the microparticle formation by blending the separately prepared microparticle batches.

8. A method as claimed in any one of the preceding claims, wherein said antigenic substance is a single bacterial antigen or a mixture of at least two bacterial antigens.

9. A method as claimed in Claim 8, wherein said antigenic substance is at least a single bacterial antigen selected from the group consisting of Neisseria gonorrhea, Mycobacterium tuberculosis, Haemophilus vaginalis, Group b Streptococcaus ecoli, Microplasma hominis, Staphylococcus aureus, Staphylococcus epidermidis, Streptococcus pyrogenes, Streptococcus mutans, Aerobacter aerogenes, Escherichia coli, Salmonella enteritidis, Pseudomonas aeruginosa, Klebsiella pneumoniae, Salmonella typhimurium, Haemophilus influenzae, Streptococcus viridans, Proteus vulgaris Shigella dysenteriae, Streptococcus Group B, Diplococcus pneumoniase, Corynebacterium Acne Types 1 and 2, Hemophilus ducrevi, Granuloma inguinale, Lymphopathia venereum, Treponema pallidum, Brucella abortus, Brucella melitensis, Brucella suis, Brucella canis, Campylobacter fetus, Campylobacter fetus intestinalis, Leptospira pomona, Listeria monocytogenes, Brucella ovis, Chlamydia psittaci, Escherichia coli, Actinobacillus equuli, Salmonella abortus ovis, Salmonella abortus equi, Corynebacterium equi, Corynebacterius pyogenes, Actinobaccilus seminis, Mycoplasma bovigenitalium, Clostridium tetani, Pseudomonas maltophiia, Streptococcus equisimili, Streptococcus dysgalactiae, Streptococcus uberis, Streptococcus bovis, Pasteurella multocida, Pasteurella haemolytica, Moraxella bovis, Actinobacillus lignieresi, Corynebacterium renale, Fusobacterium necrophorum, Bacillus cereus, Salmonella dublin, Salmonella heidleberg, Salmonella paratyphi, and Yersinia enterocolitica.

10. A method as claimed in any one of the preceding claims, wherein said mammal is a bovid and said immune regulatory factor containing substance is antibody and/or lymphokinen-containing milk.

13

EP 0 300 102 B1

**11.** A method as claimed in Claim 10, wherein said bovid is a cow.

**12.** A method as claimed in Claim 11, wherein said cow is immunized to a hyperimmunized state with an antigenic substance sufficient to produce (a) milk containing higher than normal levels of lymphokinens which include antiinflammatory factor; (b) milk containing factors which counteract debilitating conditions of the cardiovascular and pulmonary systems; (c) milk containing an antibody factor effective against dental caries, plaque and gingivitis producing bacteria; or (d) milk containing antibody factor effective against odor-causing and disease-causing skin bacteria.

**13.** A method as claimed in any one of the preceding claims, wherein said immunized state is attained by simultaneously implanting said antigenic substance containing shaped matrix material in said mammal and inoculating said mammal with a fluid containing said antigenic substance.

**Patentansprüche**

**1.** Verfahren zum Erhalten eines immunregulierenden Faktors mittels Immunisierens eines Säugetieres und Sammlung oder Gewinnung einer Substanz mit einem immunregulierenden Faktor daraus, **dadurch gekennzeichnet, daß** das Säugetier durch Implantierung einer Menge einer Antigensubstanz in das Säugetier, die ausreicht um eine Immunantwort hervorzurufen, immunisiert wird, wobei die Antigensubstanz innerhalb der Mikroteilchen aus einem bioverträglichen Matrixmaterial eingearbeitet ist, was eine gesteuerte Freisetzung der Antigensubstanz verusacht, wodurch die Antigenwirkung in dem behandelten Säugetier verlängert wird.

**2.** Verfahren nach Anspruch 1, **dadurch gekennzeichnet, daß** die Mikroteilchen in das Säugetier durch eine intramuskuläre Injektion implantiert werden.

**3.** Verfahren nach Anspruch 1, **dadurch gekennzeichnet, daß** die Mikroteilchen in das Säugetier durch eine subkutane Injektion implantiert werden.

**4.** Verfahren nach einem der vorstehenden Ansprüche, **dadurch gekennzeichnet, daß** das Matrixmaterial ein bioverträgliches, bioabbaubares polymeres Matrixmaterial ist.

**5.** Verfahren nach Anspruch 4, **dadurch gekennzeichnet, daß** das polymere Material eine Polymilchsäure, Polyglykolsäure, Poly(Milchsäure-Co-Glykolsäure), Polycaprolacton, Polyoxalsäure, Polydioxanon, Polyorthoester oder die Salzform dieser Polymere ist.

**6.** Verfahren nach einem der vorstehenden Ansprüche, **dadurch gekennzeichnet, daß** die Mikroteilchen durch Einarbeitung der Antigensubstanz(en) in eine Mischung aus mindestens zwei unterschiedlichen, bioabbaubaren und bioverträglichen Polymeren, von denen jedes eine unterschiedliche Geschwindigkeit des Bioabbaus aufweist, hergestellt werden, wodurch einzelne Mikroteilchen hergestellt werden, die aus einer Mischung aus polymeren Matrixmaterialien zusammengesetzt sind.

**7.** Verfahren nach einem der Ansprüche 1 bis 5, **dadurch gekennzeichnet, daß** die Mikroteilchen durch folgende Schritte gebildet werden:
(a) Einarbeitung der Antigensubstanz(en) in ein einziges polymeres Matrixmaterial, (b) getrenntes Wiederholen des Verfahrensschritts (a) mindestens einmal mit einem unterschiedlichen polymeren Matrixmatrial, das eine vom ersten verwendeten Matrixmaterial aus Schritt (a) unterschiedliche Geschwindigkeit des Bioabbaus aufweist, und dann (c) Vollenden der Herstellung der Mikroteilchenbildung durch Mischen der getrennt hergestellten Mikroteilchenansätze.

**8.** Verfahren nach einem der vorstehenden Ansprüche, **dadurch gekennzeichnet, daß** die Antigensubstanz ein einzelnes bakterielles Antigen oder eine Mischung aus mindestens zwei bakteriellen Antigenen ist.

**9.** Verfahren nach Anspruch 8, **dadurch gekennzeichnet, daß** die Antigensubstanz mindestens ein einzelnes bakterielles Antigen ist, ausgewählt aus der Gruppe bestehend aus: Neisseria gonorrhea, Mycobacterium tuberculosis, Haemophilus vaginalis, Gruppe b Streptococcus ecoli, Microplasma hominis, Staphylococcus aureus, Staphylococcus epidermidis, Streptococcus pyrogenes, Streptococcus

14

mutans, Aerobacter aerogenes, Escherichia coli, Salmonella enteritidis, Pseudomonas aeruginosa, Klebsiella pneumoniae, Salmonella typhimurium, Haemophilus influenzae, Streptococcus viridans, Proteus vulgaris, Shigella dysenteriae, Streptococcus Gruppe B, Diplococcus pneumoniase, Corynebacterium Acne Typen 1 und 2, Hemophilus ducreyi, Granuloma inguinale, Lymphopathia venereum, Treponema pallidum, Brucella abortus, Brucella melitensis, Brucella suis, Brucella canis, Campylobacter fetus, Campylobacter fetus intestinalis, Leptospira pomona, Listeria monocytogenos, Brucella ovis, Chlamydia psittaci, Escherichia coli, Actinobacillus equuli, Salmonella abortus ovis, Salmonella abortus equi, Corynebacterium equi, Corynebacterium pyrogenes, Actinobacillus seminis, Mycoplasma bovigenitalium, Clostridium tetani, Pseudomonas maltophiia-, Streptococcus equisimili, Streptococcus dysgalactiae, Streptococcus uberis, Streptococcus bovis, Pasteurella multocida, Pasteurella haemolytica, Moraxella bovis, Actinobacillus lignieresi, Corynebacterium renale, Fusobacterium necrophorum, Bacillus cereus, Salmonella dublin, Salmonella heidelberg, Salmonella paratyphi und Yersinia enterocolitica.

10. Verfahren nach einem der vorstehenden Ansprüche, **dadurch gekennzeichnet, daß** das Säugetier ein Bovid ist und die den immunregulierenden Faktor enthaltende Substanz eine Antikörper und/oder Lymphokin enthaltende Milch ist.

11. Verfahren nach Anspruch 10, **dadurch gekennzeichnet, daß** das Tier aus der Familie der Bovidae eine Kuh ist.

12. Verfahren nach Anspruch 11, **dadurch gekennzeichnet, daß** die Kuh zu einem hyperimmunisierten Zustand mit einer Antigensubstanz immunisiert wird, die ausreicht, um folgendes zu erzeugen: (a) Milch, die einen höheren Spiegel als normal an Lymphokinen enthält, die entzündungshemmende Faktoren einschließen; (b) Milch, die Faktoren enthält, die schwächenden Zuständen des kardiovaskulären Systems und des Lungensystems entgegenwirken; (c) Milch, die einen Antlkörperfaktor enthält, der gegen Zahnkaries, Plaque und Zahnfleischentzündung verursachende Bakterien wirksam ist; oder (d) Milch, die einen gegen Geruch oder Krankheit verursachende Hautbakterien wirksamen Antikörperfaktor enthält.

13. Verfahren nach einem der vorstehenden Ansprüche, **dadurch gekennzeichnet, daß** der Immunzustand durch das gleichzeitige Implantieren des die Antigensubstanz enthaltenden, geformten Matrixmaterials in das Säugetier und Impfen des Säugetiers mit einem die Antigensubstanz enthaltenden Fluid erreicht wird.

**Revendications**

1. Méthode d'obtention de facteurs immunorégulateurs par immunisation de mammifères et collecte ou extraction, à partir de ces mammifères, d'une substance contenant des facteurs immunorégulateurs, méthode caractérisée par le fait que l'on immunise le mammifère en implantant dans ledit mammifère une quantité d'une substance antigénique suffisante pour faire apparaitre une réponse d'immunisation, ladite substance antigénique étant incorporée dans des microparticules d'un matériau biocompatible, constituant une matrice, qui provoque la libération contrôlée de ladite substance antigénique, prolongeant ainsi l'activité antigénique dans ledit mammifère traité.

2. Méthode selon la revendication 1, dans laquelle on implante lesdites microparticules dans ledit mammifère par injection intramusculaire.

3. Méthode selon la revendication 1, dans laquelle on implante lesdites microparticules dans ledit mammifère par injection sous, cutanée.

4. Méthode selon l'une quelconque des revendications précédentes, dans laquelle le matériau constituant la matrice est un matériau polymère biocompatible, biodégradable.

5. Méthode selon l'une quelcation 4, dans laquelle ledit matériau polymère est l'acide polylactique, l'acide polyglycolique, le polyacide (acide lactique-acide co-glycolique), le polycaprolactone, l'acide polyoxalique, la polydioxanone, le polyorthoester ou la forme saline de ces polymères.

**6.** Méthode selon l'une quelconque des revendications précédentes, dans laquelle on forme les microparticules en incorporant ladite, ou lesdites substances antigéniques dans un mélange d'au moins deux polymères biodégradables, biocompatibles différents présentant chacun une vitesse différente de biodégradation, formant ainsi des microparticules individuelles composées d'un mélange de matériaux polymères constituant la matrice.

**7.** Méthode selon l'une quelconque des revendications 1 à 5, dans laquelle on forme les microparticules (a) en incorporant ladite substance antigénique, ou lesdites substances antigéniques dans un unique matériau polymère constituant une matrice, (b) en répétant séparément le processus de l'étape (a) au moins une fois avec un matériau polymère différent constituant une matrice et présentant une vitesse de biodégradation différente de celle du premier matériau, constituant la matrice, utilisé dans l'étape (a), et (c) en achevant la préparation de la formation des microparticules en mélangeant les lots de microparticules ainsi préparées séparément.

**8.** Méthode selon l'une quelconque des revendications précédentes dans laquelle ladite substance antigénique est un unique antigène bactérien ou un mélange d au moins deux antigènes bactériens.

**9.** Méthode selon la revendication 8, dans laquelle ladite substance antigénique est au moins un unique antigène bactérien choisi dans le groupe constitué de Neisseria gonorrhea, Mycobacterium tuberculosis, Haemophilus vaginalis, Group b Streptococcaus ecoli, Microplasma hominis, Staphylococcus aureus, Staphylococcus epidermidis, Streptococcus pyrogenes, Streptococcus mutans, Aerobacter aerogenes, Escherichia coli, Salmonella enteritidis, Pseudomonas aeruginosa, Klebsiella pneumoniae, Salmonella typhimurium, Haemophilus influenzae, Streptococcus viridans, Proteus vulgaris Shigella dysenteriae, Streptococcus Group B, Diplococcus pneumoniase, Corynebacterium Acne Types 1 et 2, Hemophilus ducrevi, Granuloma inguinale, Lymphopathia venereum, Treponema pallidum, Brucella abortus, Brucella melitensis, Brucella suis, Brucella canis, Campylobacter fetus, Campylobacter fetus intestinalis, Leptospira pomona, Listeria monocytogenes, Brucella ovis, Chlamydia psittaci, Escherichia coli, Actinobacillus equuli, Salmonella abortus ovis, Salmonella abortus equi, Corynebacterium equi, Corynebacterius pyogenes, Actinobaccilus seminis, Mycoplasma bovigenitalium, Clostridium tetani, Pseudomonas maltophiia, Streptococcus equisimili, Streptococcus dysgalactiae, Streptococcus uberis, Streptococcus bovis, Pasteurella multocida, Pasteurella haemolytica, Moraxella bovis, Actinobacillus lignieresi, Corynebacterium renale, Fusobacterium necrophorum, Bacillus cereus, Salmonella dublin, Salmonella heidleberg, Salmonella paratyphi, et Yersinia enterocolitica.

**10.** Méthode selon l'une quelconque des revendications précédentes, dans laquelle ledit mammifère est un bovidé et dans laquelle la substance contenant ledit facteur immunorégulateur est du lait contenant un anticorps et/ou une lymphokine.

**11.** Méthode selon la revendication 10, dans laquelle ledit bovidé est une vache.

**12.** Méthode selon la revendication 11, dans laquelle on immunise ladite vache avec une substance antigénique à un état d'hyperimmunisation suffisant pour produire (a) du lait contenant des proportions supérieures à la normale, de lymphokines qui contiennent un facteur anti-inflammatoire; (b) du lait contenant des facteurs qui s'opposent aux conditions débilitantes des systèmes cardiovasculaire et pulmonaire; (c) du lait contenant un facteur anticorps efficace contre les bactéries produisant les caries dentaires, les plaques dentaires et la gingivite; ou (d) du lait contenant un facteur anticorps efficace contre les bactéries de la peau provoquant des odeurs et des maladies.

**13.** Méthode selon l'une quelconque des revendications précédentes dans laquelle on obtient ledit état immunisé en procédant simultanément à l'implantation dans ledit mammifère, dudit matériau constituant une matrice de forme contenant la substance antigénique et à l'inoculation, audit mammifère, d'un fluide contenant ladite substance antigénique.